# EUROPEAN PATENT SPECIFICATION

(11) **EP 3 821 860 B1**
(45) Date of publication and mention of the grant of the patent: **19.06.2024**
(21) Application number: 20203663.8
(22) Date of filing: 23.10.2020
(51) Int. Cl.: A61F 13/471, A61F 13/512, A61F 13/514

(54) **ABSORBENT ARTICLE FOR MEN**
SAUGFÄHIGER ARTIKEL FÜR MÄNNER
ARTICLE ABSORBANT POUR HOMMES

(30) Priority: 12.11.2019 SE 1951303
(43) Date of publication of application: 19.05.2021
(73) Proprietor: Swereco AB, 164 40 Kista (SE)
(72) Inventor: ULFSTRÖM, Cenneth, 311 40 Falkenberg (SE)
(74) Representative: Potter Clarkson

(56) References cited:
- EP-A2- 0 861 644
- WO-A1-2012/064243
- WO-A1-2016/205756
- JP-A- 2008 136 641
- JP-A- 2010 220 835
- US-A1- 2013 006 208

## Description

### Technical field

The present invention relates generally to an absorbent article for men, which article is to be arranged in the crotch region of a man and where the main function of the absorbent article is to be used by men suffering from light incontinence or light to medium continence. More specifically, the present invention concerns an absorbent article for men which is adjustable concerning the size of a cup-like collector arranged to absorb leaking urine, to better fit different sizes of genitals.

### Background art

Incontinence and especially light incontinence are more common amongst women than amongst men whereby incontinence protection articles and absorbent articles for women are better known and there are more variants to choose from in the existing market. Due to normal anatomical differences amongst women, absorbent articles as for example incontinence protection articles for women, are over a long period of time designed and developed to fit different users. Of course, the situation amongst men is similar, with a great variety concerning anatomy in the crotch region, but the number of variants of absorbent articles for men are less compared to the number of variants absorbent articles for women.

A known male incontinence protection article is presented in WO 1992/015269. The incontinence protection article has a wider front and then tapers towards the back of the same to make it fit comfortably in the crotch region of a male user. The incontinence protection article further comprises elastic threads along the longitudinal edges of the same creating a cup-like collector which completely or partly surrounds the scrotum. This type of absorbent article is positioned only in the front region and encloses the scrotum and penis. The size of the absorbent article is not possible to adjust, and the elasticity of the product serves only as a rear leakage barrier.

JP2010207504 aims to provide a urine pad for men where the depth of the urine pad having a three-dimensional bowl-like shape is adjusted gradually according to a wearing person. The urine pad for men includes an absorbent body having an absorber, a top sheet, and a back sheet. The absorbent body has at least one pleated portion, pleated so as to overlap each other. By at least two joining parts which fix the pleated state of the pleated portions by at least two positions, the absorbent body is formed three-dimensionally so that a part of the side of a surface on which the back sheet is disposed is curved to project and a recessed space is provided on a surface on which the top sheet is disposed. By gradually releasing each fixed portion in the at least two joining parts, a part of the pleated state of the pleated portions is expanded to gradually adjust the depth of the three-dimensional shape of the absorbent body.

Another solution is presented in EP 0225909 B1, which patent discloses a method of manufacturing an absorbent article with an absorbent layer and a liquid-impermeable layer, and which is provided with two laterally separated flaps extending in the longitudinal direction of the absorbent article at one end portion of two single web portions intended as a protector. The web of material is folded together along a centerline and the flaps are brought together upon said folding and is welded together along a line extending from said point obliquely towards the free flap ends and forming an acute angle to the centerline while constituting the bottom edge of the finished protector. The connected flaps presenting a downwardly tapering, cup-like configuration. In this manner there is obtained an airy and comfortable protector, which does not encase the user's organ, but which instead forms a ventilated splash guard. Beyond being adapted to the bodily shape of the wearer, the design of the protector with a downwardly tapering, cup-like portion also provides a shield against downward splash. This solution is not possible to adjust or in any way change to fit different sizes of genitals, to fit different users.

Consequently, there is a need of an improved type of liquid-absorbent sanitary article with better possibilities to adjust the size of the same, where one product fits a number of different users.

### Summary of invention

An object of embodiments of the present invention is to provide an absorbent article for men with a possibility to adjust the size of a front part of the absorbent article and which provides a cost-efficient absorbent sanitary article which fits different sizes of genitals.

According to an aspect an absorbent article for men, which absorbent article comprising a liquid permeable top sheet facing a user's body, a liquid impermeable back sheet facing away from the user's body in a using mode of the absorbent article, and an absorbent core arranged between the top sheet and the back sheet, the absorbent article further comprising a first region arranged to cover at least a top of the user's penis and a second region arranged behind the first region, thereby extending towards a scrotum of the user in a using mode of the absorbent article, characterized in that the first region is arranged to be formed as an adjustable cup-like collector by that the first region comprises an incision arranged at a front part of the first region, which front part facing away from the second region, wherein the incision divides the front part of the first region in a left tab and a right tab, and wherein one of the left and right tabs is lockable to the other tab by that the one of the left and right tabs comprises a locking means attached to the back sheet to lock the one of the left and right tabs to the other tab; and the liquid impermeable back sheet does not comprise the incision, wherein the back sheet extends over the incision at the front part of the first region.

Consequently, the object of providing an absorbent article for men, which is adjustable concerning the size of a cup-like collector for urine absorption to better fit different sizes of genitals, is achieved. Another advantage over prior art is that the absorbent articles may be packed as flat products in their package which is advantageous, since more products may be fitted into one package which is good for transport and environment.

By letting the liquid impermeable back sheet "cover" the incision, the smooth production of the absorbent article is facilitated and the result is also that the left and right tab are kept in place in a good way both during production, during packing and before use, i.e. during the handling by the user while preparing the absorbent article for use. The latter is for example unpacking the article and setting the size by the locking means. The "cover" by the back sheet also provides a leakage protection which prevents leakage from the incision.

According to an embodiment, the locking means comprises first markings. These first markings facilitate the setting of the size of the adjustable cup-like collector, which means that a user may adjust the size in a precise manner from time to time when repeatedly using absorbent articles according to the invention. These marking may for example be simple lines, a scale with or without numbers, perforations, etc.

According to an embodiment, the back sheet comprises second markings at least at the front part of the first region. These second markings may for example be used for setting the first marking in line with one (or more) second markings, to set the correct size of the collector. For example, the first markings and the second markings may be corresponding parts of a scale or like different size steps, for example where size 1, size 2, size 3 etc. marked as first markings correspond to size 1, size 2, size 3 etc. marked as second markings. The user may for example set the locking means size 2 at the corresponding size 2 mark at the back sheet second markings. This provides an easy and repetitive sizing of the cup-like collector from the flat absorbent article with the advantages described above to a perfectly sized absorbent article for the particular user, with only one and the same absorbent article.

According to an embodiment, the first region is wider than the second region. This enables that the absorbent article is kept in place in a good way and a good protection from urine leakage around the genitals.

According to an embodiment, at least the front part of the first region has a rounded shape with a first radius, and wherein the incision extends a first distance from an outer edge of the front part in direction towards a center of the front region. To be able to freely set the size by means of the locking means arranged at one tab, the extension of the incision as well as of course the width of it, effects the possibility to set different sizes. To provide the cup-like look of the collector, the incision preferably extends at least a bit of the distance from the outer edge towards the center of the rounded front part or front region.

According to an embodiment, the first distance in which the incision extends is substantially equal to the first radius. This enables an easy setting of the collector size with an appropriate size of the incision and shape of the collector.

According to an embodiment, the first region has a substantially rounded shape.

According to an embodiment, the locking means comprises an adhesive attached to at least one of the left and right tabs. A convenient type of locking means is an adhesive which is attached to at least one of the left or the right tab and which adhesive may be covered by a release paper that is to be torn away to reveal an adhesive area of the locking means, before setting the size of the cup-like collector. When setting the size of the collector, the user may attach the adhesive area to the opposite tab in a suitable position for setting the size, preferably guided by first and/or second markings if applicable or freely if markings are not applied to the product.

According to an alternative embodiment, the locking means comprises hook and loop fastener, wherein the hook part is arranged at one of the left and right tabs and the loop part is arranged at the other of the left and right tabs which do not comprise the hook part. This is also a convenient type of locking means like the adhesive, and the setting may be facilitated within the hook and loop area of the locking means. The first and second markings may be arranged at the hook part and/or the loop part or at the back sheet or the like, if the markings exist at all at the product.

According to an embodiment, the locking means is attached to the back sheet on a side of the back sheet facing away from the absorbent core. This means that the locking means are arranged on a side facing away from the user in the using position of the absorbent article, which is to prefer wherein chafing of the skin/genitals is avoided. Since the back sheet may cover the incision, the side of the absorbent article facing the user is protected from contact with the locking means.

According to an embodiment, the at least the second region comprises an elastic part. The second region is behind the first region, seen to the using position of the absorbent article, wherein the second region is positioned in the area between the legs of the user. A "stretching area", that is the second region with the elastic part, is very comfortable for the user and suitable for remaining the absorbent article in the correct position.

According to an embodiment, the absorbent article further comprises a third region which is arranged behind the second region. Also the third region comprises the liquid permeable top sheet and the liquid impermeable back sheet and the absorbent core. The absorbent article more or less has the same layers in all three regions, and the third region is adopted by size, shape and construction to absorb any secretions from the anal of the user. By having an absorbent article with a third region, one complete product facilitates good protection from urinal leakage as well as anal leakage.

According to an embodiment, the third region comprises an odor-absorbent layer arranged between the absorbent core and the back sheet. The odor-absorbent layer may comprise a textile fabric coated with activated carbon or the like and preferably at least 90 g/m² of activated carbon. Such an absorbent article would effectively absorb both body-emitted liquids and body-emitted odors.

According to an embodiment, the liquid impermeable back sheet comprises an adhesive on the side facing away from the absorbent core, wherein the adhesive is preferably_an adhesive tape, arranged for fixing the absorbent article to a pair of underwear.

The absorbent article may also comprise a curtain-like leakage barrier extending around the edge of the absorption area on the liquid permeable top sheet, at a position at a certain distance from an outer edge of the absorbent article.

### Brief description of drawings

The invention is now described, by way of example, with reference to the accompanying drawings, in which:
Fig. 1 is a schematic top view of an absorbent article according to the invention.
Fig. 2 is a section view of the absorbent article of Fig.1.
Fig. 3 is a zoomed view from the underside of a front part of a first region of the absorbent article of Fig.1.

### Description of embodiments

In the following, a detailed description of embodiments of an absorbent article is provided.

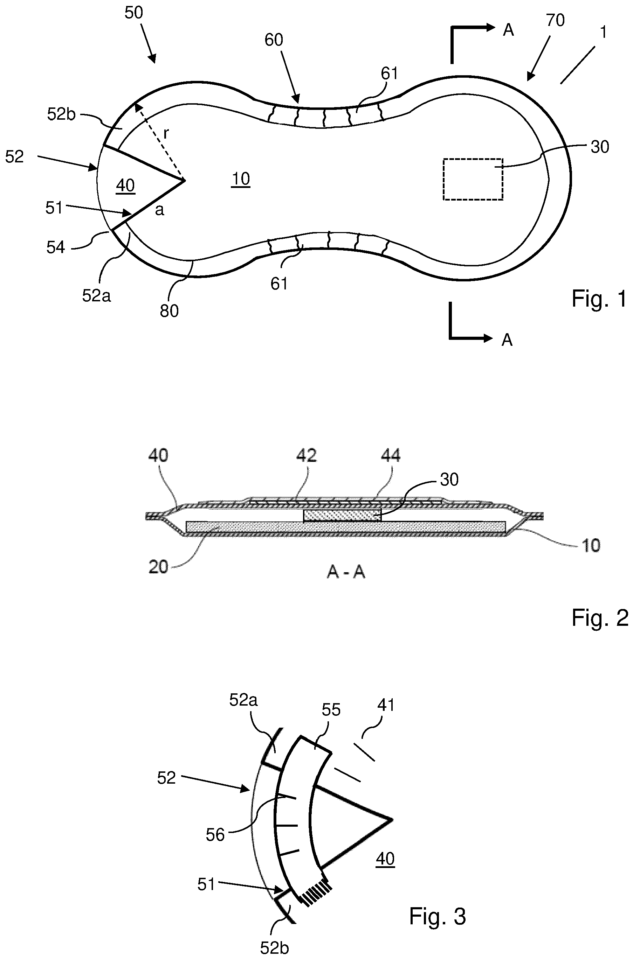

Figs. 1 shows a top view of a liquid-absorbent article 1 which comprises a liquid permeable top sheet 10, which faces a user's body and a liquid impermeable back sheet 40 which faces away from the user's body in a using mode of the absorbent article 1. Further, an absorbent core 20 is arranged between the top sheet 10 and the back sheet 40 (see further Fig. 2). The absorbent article 1 further comprises a first region 50 arranged to cover at least a top of the user's penis and an elongated second region 60 arranged behind the first region 50 and thus extending towards a scrotum of the user in the using mode of the absorbent article 1. In the preferred embodiment the absorbent article further comprises a third region 70 arranged behind the second region 60 and which third region 70 also comprises the liquid permeable top sheet 10, the liquid impermeable back sheet 40 and the absorbent core 20. By that the absorbent article 1 has the same layers/sheets 10, 20, 40 in all three regions 50, 60, 70 and the third region 70 is adopted by size, shape and construction to absorb any secretions from the anal of the user.

The first region 50 is arranged to be formed as an adjustable cup-like collector by that the first region 50 is wider than the second region 60 and preferably also has a rounded shape with a radius r. The first region 50 comprises an incision 51 arranged at a front part 52 of the first region 50. The front part 52 faces away from the second region 60, and the incision 51 divides at least the front part 52 in a left tab 52a and a right tab 52b. The incision 51 extends a first distance a from an outer edge 54 of the front part 52, in direction towards a center of the front region 50 and the first distance a in which the incision 51 extends is substantially equal to the first radius r. Other shapes of the first region 50 and other extensions of the incision 51 is also possible within the scope of the invention as long as the adjustable cup-like collector may be formed by means of the shape of the first region 50 and the incision 51 etc.

As can be seen in Fig. 1, the liquid impermeable back sheet 40 does not comprise the incision 51 wherein the back sheet 40 extends over the incision 51 at the front part 52 of the first region 50. As mentioned above, by letting the liquid impermeable back sheet 40 "cover" the incision 51, a smooth production of the absorbent article 1 is facilitated and the result is also that the left and right tabs 52a, 52b are kept in place in a good way both during production, during packing and before use, i.e. during the handling by the user while preparing the absorbent article for use. By that the back sheet 40 covers the incision 51, also a leakage protection is disclosed since any leakage of urine from the incision is prevented by the back sheet 40 covering the incision and the user is also prevented from contact with a locking means 55 attach to the back sheet 40.

In a preferred embodiment of the invention, the third region 70 comprises an odor-absorbent layer 30, which can be seen as a dashed square in Fig. 1. The size and shape of the odor-absorbent layer 30 is symbolically visualized by the dashed square and may of course vary within the scope of the invention. The odor-absorbent layer 30 is arranged between the absorbent core 20 and the back sheet 40 (see further Fig. 2).

At least the second region 60 of the absorbent article 1 may comprise an elastic part 61, which provides a "stretching area" which is very comfortable for the user and also suitable for remaining the absorbent article in the correct position. The elastic material or elastic threads or the like used in the elastic part 61 may also be applied in the first region 50 as well as in the third region 70.

The second region 60 of the absorbent article 1 may also be a part of the cup-like collector depending on how the regions 50, 60 are designed. This means that the cup-like collector may extend at least a bit into the second region 60.

Fig. 2 is a section view A - A of the absorbent article of Fig.1. Note that in the figure, the liquid permeable top sheet 10 is orientated downwards and the liquid impermeable back sheet 40 is orientated upwards. The absorbent core 20 is arranged between the top sheet 10 and the back sheet 40 and may comprise fluff and super absorbents for absorbing liquid. The top sheet 10 and the back sheet 40 are joined to each other at outer edges of the liquid-absorbent article so that the absorbent core 20 is enclosed by the joined top sheet 10 and back sheet 40. The liquid-absorbent sanitary 1 article may further comprise the odor-absorbent region 30 arranged between the absorbent core 20 and the back sheet 40. The odor-absorbent layer 30 may comprise a textile fabric coated with activated carbon, preferably at least 90 g/m² of activated carbon. Such a liquid-absorbent article 1 would effectively absorb both body-emitted liquids such as urine and anal secretions as well as body-emitted odors. The liquid-absorbent sanitary article 1 may further comprise an adhesive area 42 attached to the back sheet 40, on a side of the back sheet 40 facing away from the absorbent core 20, for attaching the absorbent sanitary 1 article to a pair of underwear. The adhesive area 42 may be covered by a release paper 44 that is to be torn away to reveal the adhesive area 42, before the absorbent sanitary article 1 is attached to a pair of underwear.

Fig. 3 is a zoomed view of the front part 52 of the first region 50 of the absorbent article 1 of Fig. 1, seen from the back sheet side 40. In the example the back sheet 40 may be of transparent material and thus the incision 51 is visible through the back sheet 40, like in the figure. According to the invention at least one of the left tab 52a and the right tab 52b is lockable to the other tab by that at least one of the left and right tabs 52a, 52b comprises a locking means 55. The locking means 55 may for example be an adhesive, preferably an adhesive tape, attached to at least one of the left and right tabs 52a, 52b wherein preferably the release paper is to be torn away to reveal an adhesive area of the locking means 55, before locking one tab to the other. For example, the right tab 52b comprises the adhesive tape 55 and may be locked to the opposite left tab 52a, to set the size of the cup-like collector formed by at least the front region 50. The setting may be at any position to the left tab 52a or on a specified area which is arranged for receiving the adhesive, like a special surface layer or the like. An alternative to adhesive is for example that the locking means 55 comprises a hook and loop fastener, wherein the hook part is arranged at one of the left and right tabs 52a, 52b and the loop part is arranged at the other of the left and right tabs 52a, 52b. The setting may be done in similar way as described above but by attaching the tab with the hook part to the tab with the loop part, in a position which corresponds to the size of the cup-like collector preferred by the user. Other locking means 55 may also be applicable such as metal or plastic hooks and corresponding metal or plastic shackles/loops, similar to for example locking means used for a bra or the like. The locking means 55 is most preferred to be attached to the back sheet 40 of the absorbent article 1, that is on the side of the back sheet 40 which faces away from the absorbent core 20.

The locking means 55 may further comprise first markings 56 which are used as an indication of how to set a proper size of the cup-like collector. The first markings 56 may be designed as simple lines or other type of markings dividing a possible "setting-length" of the locking means 55 or size of the cup-like collector. The corresponding tab 52a, 52b on which the locking means 55 is to be attached to, may comprise corresponding second markings 41 for facilitating an easy setting of one tab to the other and the setting of the size of the cup-like collector. For example, the back sheet 40 may comprise the second markings 41 at least at the front part 52 of the first region 50 or on a surface area onto which the locking means 55 is to be attached to.

## Claims

1. Absorbent article (1) for men, which absorbent article (1) comprising a liquid permeable top sheet (10) facing a user's body, a liquid impermeable back sheet (40) facing away from the user's body in a using mode of the absorbent article (1), and an absorbent core (20) arranged between the top sheet (10) and the back sheet (40), the absorbent article (1) further comprising a first region (50) arranged to cover at least a top of the user's penis and a second region (60) arranged behind the first region (50), thereby extending towards a scrotum of the user in a using mode of the absorbent article (1), **characterized in that** the first region (50) is arranged to be formed as an adjustable cup-like collector by that the first region (50) comprises an incision (51) arranged at a front part (52) of the first region (50), which front part (52) facing away from the second region (60), wherein the incision (51) divides the front part (52) of the first region (50) in a left tab (52a) and a right tab (52b), and wherein one of the left and right tabs (52a, 52b) is lockable to the other tab (52a, 52b) by that the one of the left and right tabs (52a, 52b) comprises a locking means (55) attached to the back sheet (40) to lock the one of the left and right tabs to the other tab; and the liquid impermeable back sheet (40) does not comprise the incision (51), wherein the back sheet (40) extends over the incision (51) at the front part (52) of the first region (50).

2. Absorbent article (1) according to claim 1, wherein the locking means (55) comprises first markings (56).

3. Absorbent article (1) according to any of the preceding claims, wherein the back sheet (40) comprises second markings (41) at least at the front part (52) of the first region (50).

4. Absorbent article (1) according to any of the preceding claims, wherein the first region (50) is wider than the second region (60).

5. Absorbent article (1) according to any of the preceding claims, wherein at least the front part (52) of the first region (50) has a rounded shape with a first radius (r), and wherein the incision (51) extending a first distance (a) from an outer edge (54) of the front part (52) in direction towards a center of the front region (50).

6. Absorbent article (1) according to claim 5, wherein the first distance (a) in which the incision (51) extends is substantially equal to the first radius (r).

7. Absorbent article (1) according to any of the preceding claims, wherein the first region (50) has a substantially rounded shape.

8. Absorbent article (1) according to any of the preceding claims, wherein the locking means (55) comprising an adhesive attached to at least one of the left and right tabs (52a, 52b).

9. Absorbent article (1) according to any of claims 1 - 7, wherein the locking means (55) comprising hook and loop fastener, and wherein the hook part is arranged at one of the left and right tabs (52a, 52b) and the loop part is arranged at the other of the left and right tabs (52a, 52b).

10. Absorbent article (1) according to any of the preceding claims, wherein the locking means (55) is attached to the back sheet (40) on a side of the back sheet (40) facing away from the absorbent core (20).

11. Absorbent article (1) according to any of the preceding claims, wherein at least the second region (60) comprising an elastic part (61).

12. Absorbent article (1) according to any of the preceding claims, wherein the absorbent article (1) further comprises a third region (70) which is arranged behind the second region (60) and comprising the liquid permeable top sheet (10), the liquid impermeable back sheet (40) and the absorbent core (20).

13. Absorbent article (1) according to claim 12, wherein the third region (70) comprises an odor-absorbent layer (30) arranged between the absorbent core (20) and the back sheet (40).

14. Absorbent article (1) according to any of the preceding claims, wherein the liquid impermeable back sheet (40) comprises an adhesive (42) on the side facing away from the absorbent core (20), wherein the adhesive is preferably an adhesive tape, arranged for fixing the absorbent article (1) to a pair of underwear.

## Patentansprüche

1. Saugfähiger Artikel (1) für Männer, wobei der saugfähige Artikel (1) eine flüssigkeitsdurchlässige obere Lage (10), welche dem Körper eines Benutzers zugewandt ist, eine flüssigkeitsundurchlässige hintere Lage (40), welche in einem Gebrauchsmodus des saugfähigen Artikels (1) von dem Körper des Benutzers abgewandt ist, und einen saugfähigen Kern (20), welcher zwischen der oberen Lage (10) und der hinteren Lage (40) angeordnet ist, umfasst, wobei der saugfähige Artikel (1) weiter einen ersten Bereich (50), welcher so angeordnet ist, dass er zumindest eine Spitze des Penis des Benutzers bedeckt, und einen zweiten Bereich (60) umfasst, welcher hinter dem ersten Bereich (50) angeordnet ist und sich dabei in einem Gebrauchsmodus des saugfähigen Artikels (1) zu einem Skrotum des Benutzers erstreckt, **dadurch gekennzeichnet, dass** der erste Bereich (50) so angeordnet ist, dass er als becherförmiger anpassbarer Kollektor geformt werden kann, dass der erste Bereich (50) einen Einschnitt (51) umfasst, welcher an einem vorderen Teil (52) des ersten Bereichs (50) angeordnet ist, wobei der vordere Teil (52) von dem zweiten Bereich (60) abgewandt ist, wobei der Einschnitt (51) den vorderen Teil (52) des ersten Bereichs (50) in eine linke Lasche (52a) und eine rechte Lasche (52b) unterteilt, und wobei eine von der linken und rechten Lasche (52a, 52b) mit der anderen Lasche (52a, 52b) verriegelbar ist, dass die eine von der linken und rechten Lasche (52a, 52b) ein Verriegelungsmittel (55) umfasst, welches an der hinteren Lage (40) befestigt ist, um die eine von der linken und rechten Lasche mit der anderen Lasche zu verriegeln; und die flüssigkeitsundurchlässige hintere Lage (40) den Einschnitt (51) nicht umfasst, wobei die hintere Lage (40) sich über den Einschnitt (51) in dem vorderen Teil (52) des ersten Bereichs (50) erstreckt.

2. Saugfähiger Artikel (1) nach Anspruch 1, wobei das Verriegelungsmittel (55) erste Markierungen (56) umfasst.

3. Saugfähiger Artikel (1) nach einem der vorstehenden Ansprüche, wobei die hintere Lage (40) zumindest in dem vorderen Teil (52) des ersten Bereichs (50) zweite Markierungen (41) umfasst.

4. Saugfähiger Artikel (1) nach einem der vorstehenden Ansprüche, wobei der erste Bereich (50) breiter ist als der zweite Bereich (60).

5. Saugfähiger Artikel (1) nach einem der vorstehenden Ansprüche, wobei zumindest der vordere Teil (52) des ersten Bereichs (50) eine abgerundete Form mit einem ersten Radius (r) aufweist, und wobei der Einschnitt (51) sich in einem ersten Abstand (a) von einem äußeren Rand (54) des vorderen Teils (52) in einer Richtung hin zu einem Mittelpunkt des ersten Bereichs (50) erstreckt.

6. Saugfähiger Artikel (1) nach Anspruch 5, wobei der erste Abstand (a), über den sich der Einschnitt (51) erstreckt, im Wesentlichen gleich dem ersten Radius (r) ist.

7. Saugfähiger Artikel (1) nach einem der vorstehenden Ansprüche, wobei der erste Bereich (50) eine im Wesentlichen abgerundete Form aufweist.

8. Saugfähiger Artikel (1) nach einem der vorstehenden Ansprüche, wobei das Verriegelungsmittel (55) einen Klebstoff umfasst, der an mindestens einer der linken und rechten Laschen (52a, 52b) angebracht ist.

9. Saugfähiger Artikel (1) nach einem der Ansprüche 1-7, wobei die Verriegelungsmittel (55) einen Haken- und Schlingenverschluss umfassen und wobei der Hakenteil an einer der linken und rechten Laschen (52a, 52b) und der Schlingenteil an der anderen der linken und rechten Laschen (52a, 52b) angeordnet ist.

10. Saugfähiger Artikel (1) nach einem der vorstehenden Ansprüche, wobei das Verriegelungsmittel (55) auf einer dem saugfähigen Kern (20) abgewandten Seite der hinteren Lage (40) an der hinteren Lage (40) befestigt ist.

11. Saugfähiger Artikel (1) nach einem der vorstehenden Ansprüche, wobei mindestens der zweite Bereich (60) einen elastischen Teil (61) umfasst.

12. Saugfähiger Artikel (1) nach einem der vorstehenden Ansprüche, wobei der saugfähige Artikel (1) ferner einen dritten Bereich (70) umfasst, der hinter dem zweiten Bereich (60) angeordnet ist und umfassend die flüssigkeitsdurchlässige obere Lage (10), die flüssigkeitsundurchlässige hintere Lage (40) und den saugfähigen Kern (20).

13. Saugfähiger Artikel (1) nach Anspruch 12, wobei der dritte Bereich (70) eine geruchsabsorbierende Schicht (30) umfasst, die zwischen dem saugfähigen Kern (20) und der hinteren Lage (40) angeordnet ist.

14. Saugfähiger Artikel (1) nach einem der vorstehenden Ansprüche, wobei die flüssigkeitsundurchlässige hintere Lage (40) auf der dem saugfähigen Kern (20) abgewandten Seite einen Klebstoff (42) umfasst, wobei der Klebstoff vorzugsweise ein Klebeband ist, das zur Befestigung des saugfähigen Artikels (1) an einem Paar Unterwäsche angeordnet ist.

## Revendications

1. Article absorbant (1) pour hommes, lequel article absorbant (1) comprend une feuille supérieure perméable aux liquides (10) faisant face au corps d'un utilisateur, une feuille arrière imperméable aux liquides (40) opposée au corps de l'utilisateur dans un mode d'utilisation de l'article absorbant (1), et une âme absorbante (20) agencée entre la feuille supérieure (10) et la feuille arrière (40), l'article absorbant (1) comprenant en outre une première région (50) agencée pour recouvrir au moins un sommet du pénis de l'utilisateur et une seconde région allongée (60) agencée derrière la première région (50), s'étendant ainsi vers un scrotum de l'utilisateur dans un mode d'utilisation de l'article absorbant (1), **caractérisé en ce que** la première région (50) est agencée pour être formé sous la forme d'un collecteur en forme de coupelle réglable en ce sens que la première région (50) comprend une incision (51) agencée au niveau d'une partie avant (52) de la première région (50), laquelle partie avant (52) est tournée à l'opposé de la seconde région (60), dans lequel l'incision (51) divise la partie avant (52) de la première région (50) en une languette gauche (52a) et une languette droite (52b), et dans lequel l'une des languettes gauche et droite (52a, 52b) peut être verrouillée sur l'autre languette (52a, 52b) en ce sens que l'une des languettes gauche et droite (52a, 52b) comprend un moyen de verrouillage (55) fixé à la feuille arrière (40) pour verrouiller l'une des languettes gauche et droite à l'autre languette ; et la feuille arrière imperméable aux liquides (40) ne comprend pas l'incision (51), dans lequel la feuille arrière (40) s'étend sur l'incision (51) au niveau de la partie avant (52) de la première région (50).

2. Article absorbant (1) selon la revendication 1, dans lequel le moyen de verrouillage (55) comprend des premiers marquages (56).

3. Article absorbant (1) selon l'une quelconque des revendications précédentes, dans lequel la feuille arrière (40) comprend des seconds marquages (41) au moins au niveau de la partie avant (52) de la première région (50).

4. Article absorbant (1) selon l'une quelconque des revendications précédentes, dans lequel la première région (50) est plus large que la seconde région (60).

5. Article absorbant (1) selon l'une quelconque des revendications précédentes, dans lequel au moins la partie avant (52) de la première région (50) a une forme arrondie avec un premier rayon (r), et dans lequel l'incision (51) s'étend sur une première distance (a) à partir d'un bord externe (54) de la partie avant (52) en direction d'un centre de la région avant (50).

6. Article absorbant (1) selon la revendication 5, dans lequel la première distance (a) sur laquelle s'étend l'incision (51) est sensiblement égale au premier rayon (r) .

7. Article absorbant (1) selon l'une quelconque des revendications précédentes, dans lequel la première région (50) a une forme sensiblement arrondie.

8. Article absorbant (1) selon l'une quelconque des revendications précédentes, dans lequel le moyen de verrouillage (55) comprend un adhésif fixé à au moins l'une des languettes gauche et droite (52a, 52b).

9. Article absorbant (1) selon l'une quelconque des revendications 1 à 7, dans lequel le moyen de verrouillage (55) comprend une fermeture à crochets et à boucles, et dans lequel la partie crochet est agencée au niveau de l'une des languettes gauche et droite (52a, 52b) et la partie boucle est agencée au niveau de l'autre des languettes gauche et droite (52a, 52b).

10. Article absorbant (1) selon l'une quelconque des revendications précédentes, dans lequel le moyen de verrouillage (55) est fixé à la feuille arrière (40) sur un côté de la feuille arrière (40) opposé à l'âme absorbante (20).

11. Article absorbant (1) selon l'une quelconque des revendications précédentes, dans lequel au moins la seconde région (60) comprend une partie élastique (61).

12. Article absorbant (1) selon l'une quelconque des revendications précédentes, dans lequel l'article absorbant (1) comprend en outre une troisième région (70) qui est agencée derrière la deuxième région (60) et comprenant la feuille supérieure perméable aux liquides (10), la une feuille arrière imperméable aux liquides (40) et l'âme absorbante (20).

13. Article absorbant (1) selon la revendication 12, dans lequel la troisième région (70) comprend une couche absorbant les odeurs (30) agencée entre l'âme absorbante (20) et la feuille arrière (40).

14. Article absorbant (1) selon l'une quelconque des revendications précédentes, dans lequel la feuille arrière imperméable aux liquides (40) comprend un adhésif (42) sur le côté opposé à l'âme absorbante (20), dans lequel l'adhésif est de préférence un ruban adhésif, agencé pour fixer l'article absorbant (1) à une paire de sous-vêtements.
